# EUROPEAN PATENT APPLICATION

(11) **EP 4 631 567 A1**
(43) Date of publication of application: **15.10.2025**
(21) Application number: 25169529.2
(22) Date of filing: 09.04.2025
(51) Int. Cl.: A61N 1/39, A61B 5/352, A61B 5/361

(54) **DEFIBRILLATOR UNIT SETTING AN AMPLITUDE OF DEFIBRILLATION SHOCK**

(30) Priority: 12.04.2024 US 202463633126 P
(71) Applicant: Georg-August-Universität Göttingen Stiftung Öffentlichen Rechts, Universitätsmedizin, 37075 Göttingen (DE); Deutsches Primatenzentrum GmbH, 37077 Goettingen (DE)
(72) Inventor: WITT, Annette, 37075 Göttingen (DE); RICHTER, Claudia, 37077 Göttingen (DE)
(74) Representative: Gerstein, Hans Joachim

(57) **Abstract**

A method for setting an amplitude of defibrillation shock as a function of parameters of an electrocardiogram (ECG) of a patient is disclosed. The method comprises the steps of determining an index of non-regularity of fibrillation and the frequency of the fibrillating heart, and setting the amplitude for shock defibrillation to a stored maximum energy (Eₘₐₓ) in case that the index of non-regularity (I_{IR}) is equal or above a given threshold value (θ_{IR}) or setting the amplitude for shock defibrillation to a value determined by a function of the frequency of the fibrillating heart (f) and the index of non-regularity (I_{IR}).

## Description

The invention relates to an optimized amplitude defibrillation (OptiAmp). A new termination-protocol for pulseless ventricular tachyarrhythmia (pVT) using external and internal defibrillators is presented.

In the European Union each year approximately 250,000 patients are dying due to sudden cardiac death (SCD, Empana et al., 2022), which is mainly based on a sudden pumping malfunction. SCD is often preceded by sudden cardiac arrhythmia known as pulseless ventricular tachyarrhythmia (pVT). These arrhythmia are characterized by abnormal patterned electrocardiograms (ECG) that do not consist of the sinus rhythm typical recurring electrophysiological signal sequence (i.e., successions of p-wave, QRS complex and t-wave), but instead show rapid oscillations with various grades of complexity. To prevent the severe consequences of pVT, such as full-bodied oxygen deprivation, immediate arrhythmia termination is crucial. The current clinical practice involves delivering high-energy electrical shocks using external as well as implanted cardioverter/defibrillators. In reality, multiple defibrillation attempts are oftentimes necessary to successfully terminate the arrhythmia. Against this background, it should not go unmentioned that each application of high-energetic electrical shocks causes cellular processes potentially leading to the destruction of heart tissue. Here, higher shock amplitudes and higher number of shocks equal in accumulation of structural damage ultimately resulting in irreversible tissue remodelling. This process itself is leading to a reduction in heart function and consequently a deterioration in the overall prognosis for the patient. Furthermore, with an increasing instability in physical health status patients also often experience psychological suffering manifesting for example in anxiety and depression (Sola & Bostwick, 2005). Therefore, reducing the number of defibrillation shocks is of high importance not only for health political issues.

Currently, two standard strategies are used for defibrillating pVTs:
A. Defibrillation attempts with shocks of identical amplitude are delivered (max. 360 J for external defibrillators (e.g. by the Corpuls "corpuls3" device), max. 42 J for implantable cardioverter defibrillators (ICD) and max. 80 J for subcutaneous ICD (s-ICD)) are repeated until defibrillation is successful, or,
B. the amplitude of the shocks is increased (e.g. for external defibrillation with the Stryker "LIFEPAK^{®} 15": 1st shock with 200 J, 2nd shock with 300 J and 3rd shock with 360 J).

An object of the present invention is to provide an improved method for setting an amplitude of defibrillation shock and a respective defibrillator device.

The object is achieved by the method comprising the features of claims 1 and the defibrillator comprising the features of claims 8. Preferred embodiments are described in the dependent claims and the description.

The method for setting an amplitude of defibrillation shock as a function of parameters of an electrocardiogram ECG of a patient comprises the steps of determining an index of non-regularity of fibrillation and the frequency of the fibrillating heart, and setting the amplitude for shock defibrillation to a stored maximum energy in case that the index of non-regularity is equal or above a given threshold value or setting the amplitude for shock defibrillation to a value determined by a function of the frequency of the fibrillating heart and the index of non-regularity.

The present invention is based on performing series of in vivo and ex vivo defibrillation experiments on animal models (Oryctolagus cuniculus (rabbit, here: New Zealand White), Sus scrofa f. domestica (Göttingen minipig, landrace pig)) and observing that:
A. in general, regular pVT can be terminated with lower shock energies than irregular pVT.
B. in general, slow pVT can be terminated with lower shock energies than fast pVT.
C. in general, irregular pVT can only be terminated with high shock energies, and
D. failed defibrillation attempts lead to faster and more irregular pVT.

Based on these observations, it is proposed by the present invention to determine the amplitude of the defibrillation shock as a function of electrocardiogram ECG parameters that characterize
(i) the non-regularity of the fibrillation and
(ii) the frequency of the fibrillating heart.

The ECG parameters are preferably determined from a digital ECG signal. This digital ECG signal is preferably preprocessed in order to reduce the effects of artifacts, such as motion artifacts, noise and all other components which are not caused by electro-mechanical processes of the considered heart. These effects of artifacts in the digital ECG signal can be removed, e.g. by digital filtering.

The digital ECG signal can be inverted, e.g. by multiplying the ECG signal with a factor of -1 such that the R-peaks point upwards.

For each fibrillation, the algorithm determines an index (i.e., a number I_{IR}) that describes its non-regularity. If this index I_{IR} is sufficiently small (i.e. smaller than a threshold value, I_{IR} < *θ*_{IR}), then a fibrillation frequency f can be determined, otherwise we call the fibrillation irregular.

The amplitude of the defibrillation shock is the amplitude chosen as follows:
A. In case of a non-regular fibrillation (i.e., I_{IR} ≥ *θ*_{IR}) the maximum shock amplitude is applied.
B. For regular fibrillations (i.e., the corresponding ECG shows essentially periodic oscillations, I_{IR} < *θ*_{IR}), the shock amplitude depends on the fibrillation frequency f and the non-regularity index I_{IR}:
   - Slow and regular fibrillations can be terminated with comparably low shock amplitudes.
   - Fibrillation frequencies in an intermediate range can be terminated with moderate shock amplitudes.
   - High fibrillation frequencies require high shock amplitudes.

The described Optimized Amplitude Defibrillation responds to the patient's current individual cardiac problems. The implementation of Optimized Amplitude Defibrillation leads to a significant reduction of number of defibrillation attempts.

The fibrillation frequency, i.e. the frequency of the fibrillating heart, can be determined as the inverse value of the center of the distribution represented by the time intervals between succeeding maxima of the electrocardiogram signal. The center of the distribution can be evaluated as
(a) the mean value of the time intervals,
(b) the median value (e.g., by determining the 50th percentile) of the time intervals or
(c) a quantile of the distribution the time intervals that is close to the median, such as, for example, the 40th or the 60th percentile.

Outlier extraction might be applied beforehand.

The method can be performed based directly on an original electrocardiogram ECG taken from a patient, or, based on a signal derived from the original electrocardiogram, in particular from the power spectrum of the original electrocardiogram. Thus, the fibrillation frequency, i.e. the frequency of the fibrillating heart, can be estimated from the signal derived from the original electrocardiogram. Accordingly, the index of non-regularity can be determined as a function of parameters of the signal derived from the original electrocardiogram, e.g. as a function of power spectrum parameters.

The present invention can be implemented by a software update for existing implantable cardioverter-defibrillators and for automated external defibrillators. Automated external defibrillators might need to completed by a signal processing unit.

Below, it is explained how the non-regularity index I_{IR} can be computed by from a digitalized ECG:
The peaks of the ECG can be detected with an appropriate peak finding algorithm that incorporates the refractory period, which identifies in case of the sine rhythm the R-peaks and in case of pVTs local maxima. A potential algorithm is described in Bruggemann et al. (2016).

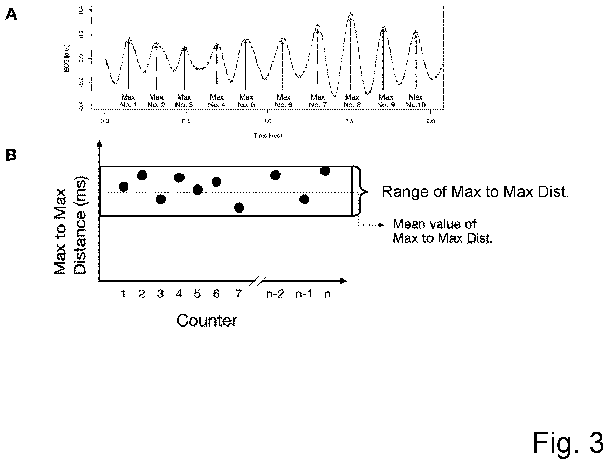

The time intervals between succeeding maxima can be statistical evaluated. An appropriate number (n = 8, ..., 50) of such intervals is considered. The non-regularity of the fibrillation, I_{IR}, can be quantified by the following quantities:
(a) the range of the intervals (see Figure 3B),
(b) the ratio of range and mean interval lengths,
(c) the variance of the intervals,
(d) the coefficient of variation (ratio of standard deviation and mean value of the interval lengths), or
(e) the range of amplitudes of the maximum peaks,
(f) the ratio of range and mean amplitudes of the maximum peaks,
(g) the variance of the amplitudes of the maximum peaks,
(h) the coefficient of variation (ratio of standard deviation and mean value of the amplitudes of the maximum peaks).

The set can be split into two sets, i.e., the time interval with even and with odd indices. All quantities which are defined below can be computed for both sets, and finally the average of the two quantities can be considered as result.

It might be advisable to remove outliers from the set of time intervals, for instance, by removing the smallest and the largest time interval.

Instead of the proposed n = 3 different shock amplitudes for defibrillating the tachyarrhythmia, other number of shock amplitudes (n = 2 or n ≥ 4) can be used. It is also possible to use a shock amplitude that continuously depends on the frequency f and the non-regularity index I_{IR} which are both measured instantaneously from the electrocardiogram ECG.

The proposed Optimized Amplitude Defibrillation protocol for terminating pVT is described above for single-pulse defibrillation protocols. Optimized amplitude defibrillation can also be applied to multi-pulse defibrillation protocols (as for example LEAP (Luther at al., 2011), adaptive deceleration pulsing (Lilienthal et al. 2022) und Janardhan et al. (2012)), the function that describes the dependence of the pulse amplitudes on the fibrillation frequency and non-regularity have to be adapted.

The invention is exemplarily described by a preferred embodiment in the enclosed figures. It shows:

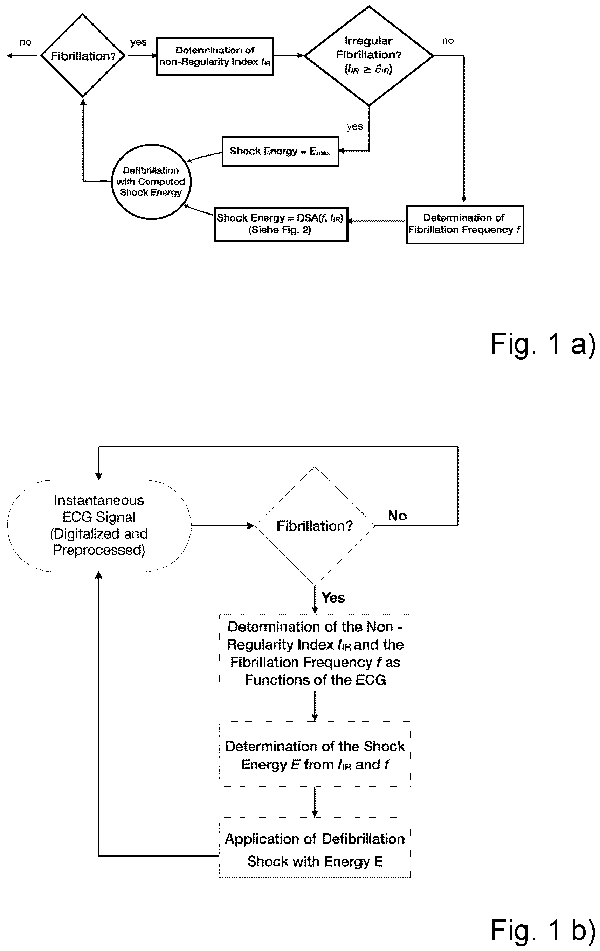

- Figure 1 a):: Schematic of Optimized Amplitude Defibrillation (DSA: Defibrillation Shock Amplitude);
- Figure 1 b):: Detailed Schematic flow of Optimized Amplitude Defibrillation (DSA: Defibrillation Shock Amplitude);
- Figure 2:: Determination of the defibrillation shock amplitude based on fibrillation frequency and non-regularity index;
- Figure 3:: Schematic for the determination of the non-regularity index I_{IR}

Figure 1 a) presents a schematic flow diagram for Optimized Amplitude Defibrillation (DSA: Defibrillation Shock Amplitude).

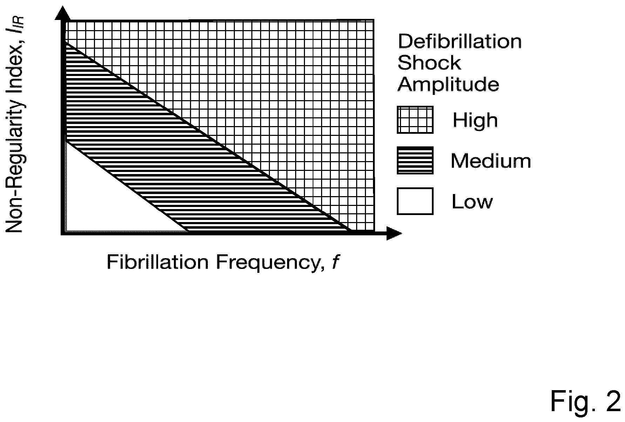

In a first step it is determined is fibrillation is needed. In case that fibrillation is required, a non-Regularity index I_{IR} is determined in a second step. In a third step, the need of irregular fibrillation is considered in case the non-regularity index I_{IR} is equal of larger than a given threshold value *θ*_{IR}. In case of irregular fibrillation, the shock energy is set to a maximum shock energy Emax. Otherwise, the fibrillation frequency f is determined and the shock energy is set to a value calculated as function of the fibrillation frequency f and the non-regularity index I_{IR} according to Figure 2.

Finally, defibrillation is performed with the computed shock energy.

Figure 1 b) presents a detailed schematic flow diagram based on Figure 1 a) for Optimized Amplitude Defibrillation (DSA: Defibrillation Shock Amplitude).

As a starting point, instantaneous electrocardiogram ECG signals are digitalized and preprocessed. Based on these measurement data, in a first decision step it is determined is fibrillation is needed. In case that fibrillation is required ("Yes"), a non-Regularity index I_{IR} and the fibrillation frequency f as function of the electrocardiogram ECG is determined in a second step. In a third step, the shock energy E is determined as function of the non-Regularity index I_{IR} and the fibrillation frequency f. Then, in a fourth step, defibrillation is applied with the computed shock energy E. After defibrillation, and also in case that no need of fibrillation is determined, a following instantaneous ECG signal is measured, digitalized and preprocessed.

Preprocessing of the digital ECG signal is provided to remove e.g. all artifacts such as motion artifacts, noise and other signal components which are not caused by electro-mechanical processes of the considered heart. The preprocessed ECG signal can be inverted, i.e. multiplied by the factor of -1, such that the R-peaks point upwards.

Figure 2 shows a diagram of the function of the non-regularity index I_{IR} and the fibrillation frequency f for determination of the defibrillation shock amplitude based on fibrillation frequency f and non-regularity index I_{IR}. The defibrillation shock amplitude is set to a low value in case that the non-regularity index I_{IR} and the fibrillation frequency f is below a lower linear curve. The defibrillation shock amplitude is set to a medium value in case that the non-regularity index I_{IR} and the fibrillation frequency f is above the lower linear curve and below an upper falling linear curve, wherein the lower and upper parallel curve are parallel to each other and have a decreasing non-regularity index value I_{IR} with increasing fibrillation frequency f (i.e. falling curve). The defibrillation shock amplitude is set to a high value in case that the non-regularity index I_{IR} and the fibrillation frequency f is above the upper linear curve.

The defibrillation shock amplitude is based on thresholding of a function that depends on at least two variables, including the non-regularity index and the fibrillation frequency. This function should be essentially monotonic in these two variables, i.e. growing (decreasing) values of the non-regularity index and the fibrillation frequency should lead to (decreasing) growing values of this function. A potential function is a linear function, i.e., a weighted sum of the non-regularity index and the fibrillation frequency and an offset. A series of thresholds can be defined. Values of the function between two succeeding thresholds or equal to one of the thresholds can be assigned to a specific shock amplitude.

Even if the function comprises small ripples, this is sufficiently monotonic. In case that the function is nonlinear, the diagram in figure 2 would have two curves instead of straight lines between the low and medium defibrillation shock amplitude region and between the medium and high defibrillation shock amplitude region.

Figure 3 shows a schematic for the determination of the non-regularity index I_{IR}. The ECG of a patient is measured over the time t and peaks (maximum values) of the ECG signal are determined. The time between subsequent maximum values (peaks) is determined, i.e. the Max to Max distance in milliseconds [ms]. For a number of peaks, a range of Max to Max distances is provided. A mean value of the Max to Max distances can be calculated from the number of Max to Max distances.

The non-regularity of the fibrillation, I_{IR}, can be quantified at least one of the following quantities selected from the group of:
(a) the range of the intervals (see Figure 3B),
(b) the ratio of range and mean interval lengths,
(c) the variance the intervals, and/or
(d) the coefficient of variation (ratio of standard deviation and mean value of the interval lengths).

### References:

1) Empana JP et al.: Incidence of Sudden Cardiac Death in the European Union, Journal of the American College of Cardiology, 79 1818-1827 (2022), doi: 10.1016/j.jacc.2022.02.041.
2) Sola, CL & Bostwick, JM: Implantable cardioverter-defibrillators, induced anxiety, and quality of life. Mayo Clinic Proceedings. Elsevier, 80(2) 232-237 (2005), doi: 10.4065/80.2.232
3) Brüggemann T et al.: Tachycardia detection in modern implantable cardioverter-defibrillators. Herzschrittmacherther Elektrophysiol. 27 171-85 (2016), doi: 10.1007/s00399-016-0449-z. Epub 2016
4) Luther S, Fenton FH et al.: Low-energy control of electrical turbulence in the heart. Nature 475 235-9 (2011) doi: 10.1038/nature10216.
5) Lilienkamp T et al.: Taming cardiac arrhythmias: Terminating spiral wave chaos by adaptive deceleration pacing. Chaos 32 (12) (2022): 121105. https://doi.org/10.1063/5.0126682
6) Janardhan, AH et al.: A Novel Low-Energy Electrotherapy That Terminates Ventricular Tachycardia With Lower Energy Than a Biphasic Shock When Antitachycardia Pacing Fails, Journal of the American College of Cardiology, 60, 2393-2398 (2012), https://doi.org/10.1016/j.jacc.2012.08.1001.
7) Empfehlung 01/4-2020 vom 17.11.2020 des Rettungsdienstausschuss Bayern: Empfehlung für die Wahl der Energie (Joule) für Defibrillation und Kardioversion im Rettungsdienst Bayern erarbeitet durch AG 4 - Patientenversorgung und Hygiene

## Claims

1. Method for setting an amplitude of defibrillation shock as a function of parameters of an electrocardiogram (ECG) of a patient comprising the steps of determining an index of non-regularity of fibrillation and the frequency of the fibrillating heart, and setting the amplitude for shock defibrillation to a stored maximum energy (Eₘₐₓ) in case that the index of non-regularity (I_{IR}) is equal or above a given threshold value (θ_{IR}) or setting the amplitude for shock defibrillation to a value determined by a function of the frequency of the fibrillating heart (f) and the index of non-regularity (I_{IR}).

2. Method according to claim 1, **characterized in** preprocessing a digital ECG signal of the electrocardiogram (ECG) in order to reduce the effects of artifacts in the digital ECG signal which are not caused by electro-mechanical processes of the considered heart and determining the parameters of the electrocardiogram (ECG) with the preprocessed digital ECG signal.

3. Method according to claim 2, **characterized by** inverting the digital ECG signal in the step of preprocessing.

4. Method according to one of the preceding claims 1 to 3, wherein the non-regularity index of the fibrillation (I_{IR}) is determined by at least one of the quantities selected from the group of:
(a) the range of the intervals between maximum peaks in the electrocardiogram (ECG) of the patient,
(b) the standard deviation of the intervals between maximum peaks in the electrocardiogram (ECG) of the patient,
(c) the difference between two quantiles of the intervals between maximum peaks in the electrocardiogram (ECG) of the patient, as for instance, the interquartile range (i.e., the difference between the 75th and the 25th percentile) or the difference between the 80th and 20th percentile or between the 90th and 10^{th} percentile,
(d) the ratio of the range and the mean or median value of the interval lengths between the maximum peaks in the electrocardiogram (ECG) of the patient,
(e) the ratio of the standard deviation of the intervals between maximum peaks in the electrocardiogram (ECG) of the patient and the mean or median value of the interval lengths between the maximum peaks in the electrocardiogram (ECG) of the patient,
(f) the ratio of the difference of two quantiles of the intervals between maximum peaks in the electrocardiogram (ECG) of the patient, as for instance, the interquartile range (i.e., the difference between the 75th and the 25th percentile) or the difference between the 80th and 20th percentile or between the 90th and 10th percentile, and the mean or median value of the interval lengths between the maximum peaks in the electrocardiogram (ECG) of the patient,
(g) the coefficient of variation of the amplitudes of the maximum peaks in the electrocardiogram (ECG) of the patient,
(h) the range of the amplitudes of the maximum peaks in the electrocardiogram (ECG) of the patient,
(i) the standard deviation of the amplitudes of the maximum peaks in the electrocardiogram (ECG) of the patient,
(j) the difference between two quantiles of the amplitudes of the maximum peaks in the electrocardiogram (ECG) of the patient, as for instance, the interquartile range (i.e., the difference between the 75th and the 25th percentile) or the difference between the 80th and 20th percentile or between the 90th and 10^{th} percentile,
(k) the ratio of the range and the mean or median value of the amplitudes of the maximum peaks in the electrocardiogram (ECG) of the patient,
(l) the ratio of the standard deviation of the amplitudes of the maximum peaks in the electrocardiogram (ECG) of the patient and the mean or median value of the amplitudes of the maximum peaks in the electrocardiogram (ECG) of the patient,
(m) the ratio of the difference of two quantiles of the amplitudes of the maximum peaks in the electrocardiogram (ECG) of the patient, as for instance, the interquartile range (i.e., the difference between the 75th and the 25th percentile) or the difference between the 80th and 20th percentile or between the 90th and 10th percentile, and the mean or median value of the amplitudes of the maximum peaks in the electrocardiogram (ECG) of the patient.

5. Method according to claim 4, wherein the coefficient of variation is determined as the ratio of standard deviation and/or the mean or median value of the interval lengths of the maximum peaks in the electrocardiogram (ECG) of the patient.

6. Method according to claim 4, wherein the coefficient of variation is determined as the ratio of standard deviation and/or the mean or median value of the amplitudes of the maximum peaks in the electrocardiogram (ECG) of the patient.

7. Method according to one of the preceding claims, **characterized in, that** the fibrillation frequency is determined as the inverse value of the center of the distribution represented by the time intervals between succeeding maxima of the electrocardiogram (ECG).

8. Method according to claim 7, **characterized by** evaluating the center of distribution as
a) the mean value of the time intervals;
b) the medial value of the time intervals;
c) a quantile of the distribution of the time intervals that are in the range of the median.

9. Method according to one of the preceding claims, **characterized by** setting the amplitude of defibrillation shock as a function of a signal derived from an original electrocardiogram (ECG) taken from a patient, in particular from the power spectrum of the original electrocardiogram (ECG).

10. Defibrillator device comprising a control unit, wherein the control unit is arranged to set an amplitude of defibrillation shock as a function of parameters of an electrocardiogram (ECG) of a patient by determining an index of non-regularity of fibrillation and the frequency of the fibrillating heart, and setting the amplitude for shock defibrillation to a stored maximum energy (Eₘₐₓ) in case that the index of non-regularity (I_{IR}) is equal or above a given threshold value (θ_{IR}) or setting the amplitude for shock defibrillation to a value determined by a function of the frequency of the fibrillating heart (f) and the index of non-regularity (I_{IR}).

11. Defibrillator device according to claim 10, wherein the control unit is arranged to determine the non-regularity index of the fibrillation (I_{IR}) by at least one of the quantities selected from the group of:
(a) the range of the intervals between maximum peaks in the electrocardiogram (ECG) of the patient,
(b) the standard deviation of the intervals between maximum peaks in the electrocardiogram (ECG) of the patient,
(c) the difference between two quantiles of the intervals between maximum peaks in the electrocardiogram (ECG) of the patient, as for instance, the interquartile range (i.e., the difference between the 75th and the 25th percentile) or the difference between the 80th and 20th percentile or between the 90th and 10th percentile,
(d) the ratio of the range and the mean or median value of the interval lengths between the maximum peaks in the electrocardiogram (ECG) of the patient,
(e) the ratio of the standard deviation of the intervals between maximum peaks in the electrocardiogram (ECG) of the patient and the mean or median value of the interval lengths between the maximum peaks in the electrocardiogram (ECG) of the patient,
(f) the ratio of the difference of two quantiles of the intervals between maximum peaks in the electrocardiogram (ECG) of the patient, as for instance, the interquartile range (i.e., the difference between the 75th and the 25th percentile) or the difference between the 80th and 20th percentile or between the 90th and 10th percentile, and the mean or median value of the interval lengths between the maximum peaks in the electrocardiogram (ECG) of the patient,
(g) the coefficient of variation of the amplitudes of the maximum peaks in the electrocardiogram (ECG) of the patient,
(h) the range of the amplitudes of the maximum peaks in the electrocardiogram (ECG) of the patient,
(i) the standard deviation of the amplitudes of the maximum peaks in the electrocardiogram (ECG) of the patient,
(j) the difference between two quantiles of the amplitudes of the maximum peaks in the electrocardiogram (ECG) of the patient, as for instance, the interquartile range (i.e., the difference between the 75th and the 25th percentile) or the difference between the 80th and 20th percentile or between the 90th and 10th percentile,
(k) the ratio of the range and the mean or median value of the amplitudes of the maximum peaks in the electrocardiogram (ECG) of the patient,
(l) the ratio of the standard deviation of the amplitudes of the maximum peaks in the electrocardiogram (ECG) of the patient and the mean or median value of the amplitudes of the maximum peaks in the electrocardiogram (ECG) of the patient,
(m) the ratio of the difference of two quantiles of the amplitudes of the maximum peaks in the electrocardiogram (ECG) of the patient, as for instance, the interquartile range (i.e., the difference between the 75th and the 25th percentile) or the difference between the 80th and 20th percentile or between the 90th and 10th percentile, and the mean or median value of the amplitudes of the maximum peaks in the electrocardiogram (ECG) of the patient.

12. Defibrillator device according to claim 11, wherein the control unit is arranged to determine the coefficient of variation as the ratio of standard deviation and/or the mean value of the interval lengths between the maximum peaks in the electrocardiogram (ECG) of the patient and/or the mean or median value of the amplitudes of the maximum peaks in the electrocardiogram (ECG) of the patient.

13. Defibrillator device according to one of the preceding claims, **characterized in, that** the control unit is arranged to determine the fibrillation frequency as the inverse value of the center of the distribution represented by the time intervals between succeeding maxima of the electrocardiogram (ECG).

14. Defibrillator device according to claim 13, **characterized in, that** the control unit is arranged to evaluate the center of distribution as
d) the mean value of the time intervals;
e) the medial value of the time intervals;
f) a quantile of the distribution of the time intervals that are in the range of the median.

15. Defibrillator device according to one of claims 10 to 14, **characterized in, that** the control unit is arranged for seting the amplitude of defibrillation shock as a function of a signal derived from an original electrocardiogram (ECG) taken from a patient, in particular from the power spectrum of the original electrocardiogram (ECG).
